(19) Europäisches Patentamt
European Patent Office
Office européen des brevets

(11) **EP 3 495 813 B1**

(12) **EUROPEAN PATENT SPECIFICATION**

(45) Date of publication and mention
of the grant of the patent:
**14.09.2022 Bulletin 2022/37**

(21) Application number: **17205584.0**

(22) Date of filing: **06.12.2017**

(51) International Patent Classification (IPC):
**G01N 33/00** *(2006.01)*    **G08B 17/117** *(2006.01)*

(52) Cooperative Patent Classification (CPC):
**G01N 33/0004; G01N 33/0047; G08B 17/117**

(54) **METHOD TO DISTINGUISH INDOOR VOC SOURCES AND TO ESTIMATE HUMAN GENERATED CO2 CONCENTRATION**

VERFAHREN ZUR UNTERSCHEIDUNG VON VOC-QUELLEN IN INNENRÄUMEN UND ZUR SCHÄTZUNG DER DURCH MENSCHEN HERVORGERUFENER CO2-KONZENTRATION

PROCÉDÉ POUR DISTINGUER LES SOURCES DE COV INTÉRIEURS ET POUR ESTIMER LA CONCENTRATION DE CO2 GÉNÉRÉE PAR L'HOMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.06.2019 Bulletin 2019/24**

(73) Proprietor: **IDT Europe GmbH**
**01109 Dresden (DE)**

(72) Inventors:
• **SCHREIBER, Ronald**
**01705 Freital (DE)**
• **MEYER, Christian**
**01069 Dresden (DE)**

(74) Representative: **Lippert Stachow Patentanwälte Rechtsanwälte**
**Partnerschaft mbB**
**Krenkelstraße 3**
**01309 Dresden (DE)**

(56) References cited:
**US-B1- 6 344 798**

• **DATABASE WPI Week 201422 Thomson Scientific, London, GB; AN 2014-E49049 XP002781371, & KR 2014 0025701 A (UNIV KOOKMIN IND ACAD COOP FOUND) 5 March 2014 (2014-03-05)**
• **CAROLIN RÖSCH ET AL: "Relationship between sources and patterns of VOCs in indoor air", ATMOSPHERIC POLLUTION RESEARCH, vol. 5, no. 1, 1 January 2014 (2014-01-01) , pages 129-137, XP055478586, ISSN: 1309-1042, DOI: 10.5094/APR.2014.016**

**Description**

The invention relates to a method to distinguish indoor VOC sources and to estimate human generated Carbon Dioxide ($CO_2$) concentration.

$CO_2$ in the air has a bad influence on human productivity.

Negative effects on human body are dizziness, weakness, dyspnea, unconsciousness up to death.

It is so dangerous because $CO_2$ has no smell and no visible color; therefore it is not detectable by human beings without any instrumentation.

$CO_2$ is generated by humans, animals and plants by breathing on the one side; and on the other side it is generated by artificial sources like fire, carbonated drinks, etc.

The knowledge of the actual $CO_2$ concentration indoors can therefore enhance some applications; for instance automated room ventilation can be switched on if the concentration of $CO_2$ gets too high.

It is well-known that $CO_2$ concentration can be measured with $CO_2$ sensitive sensors. Known examples are spectroscopic sensors (NDIR), but these are expensive and large. Also chemical sensors can be used, but these types of sensors often have a high drift and provide only a short lifetime.

US6,344,798 discloses a simulation model employing a temporal carbon dioxide rate of change calculation methodology which can differentiate between human and non-human CO2 sources in an indoor environment leading to proper decision making in relation to ventilation issues.

Some sources of $CO_2$, like humans and animals, generate VOCs proportional to $CO_2$ as well. VOCs are known as Volatile Organic Compounds, whereas VOC is often used for a single gas. If there is a mixture of VOC gases the abbreviation TVOC will be used, which means total VOC. If someone comes into a meeting room and smells an uncomfortable smell of the exhausted air, this is often caused by the VOC contents.

It is also well-known that VOC sensitive sensors, like metal oxide gas sensors (MOX gas sensors), are used to measure VOC. These sensors can also be used to estimate the $CO_2$ concentration based on the assumption that $CO_2$ and VOC are generated in a proportional way. It should be noted that $CO_2$ is more common to most people than VOC. In order to distinguish between an estimated and a true $CO_2$ concentration, the name "$eCO_2$" is used for the estimated $CO_2$ concentration.

In the state-of-the-art a MOX sensor is used to measure the VOC and calculate at the same time the estimation of human generated $CO_2$ concentration ($eCO_2$) based on the VOC measurement. But such a measurement is vague since it is based on the assumption that $CO_2$ by human or animals is generated proportionally to VOC, whereas the following equation is used to estimate $CO_2$ concentration:

$$eCO_2 = a + b \cdot VOC \qquad\qquad (\text{eq. 1})$$

whereas a and b are constant coefficients, b is the ratio between the human generated $CO_2$ and VOC, a is a constant offset. Sometimes linear filtering is used in addition to reduce noise or to remove spikes and notches.

But in real scenarios and, in reality, there are indoor VOC sources which do not generate $CO_2$ in the same way as humans or animals, for instance deodorants, air freshener, white board cleaner, food preparation, etc. These examples only generate VOC and do not contribute to a $CO_2$ increase. Therefore, the assumption used for existing $eCO_2$ technology is not complete and eq. 1 will lead to large errors in the estimated $CO_2$ concentration. Also, linear low-pass filtering cannot suppress these VOC sources.

It is therefore the object of this invention to improve the estimated $CO_2$ ($eCO_2$) concentration for non-industrial indoor applications.

The accuracy of the estimation of $CO_2$ can be improved by exploiting the following boundary conditions: First, the $CO_2$ concentration in the earth's atmosphere is about 400ppm which means that an indoor concentration cannot be lower and hence can be seen as a natural lower concentration limit. Second, it is proved that concentrations above 5000ppm are extremely unlikely at home or indoors.

Based on these known boundary conditions it can be deduced from differences in temporal characteristics that a decrease in VOC concentrations indicates the existence of ventilation. Assuming that the human is not in an uncomfortable small room the human generated VOC as well as the human generated $CO_2$ can only rise slowly. Hence, a rapid increase in VOC concentrations must be caused by non-human sources.

The object of the invention will be solved by a method in accordance with independent claim 1 to improve estimated $CO_2$ ($eCO_2$) concentration for indoor applications, comprising the following steps:

- observing a temporal change of a VOC concentration by measuring a present VOC concentration and comparing the result with a previous VOC concentration of a previous measurement of the VOC concentration,
- splitting the change of the VOC concentration into a human generated VOC concentration change and a non-human generated VOC concentration change,

- accumulate the human generated VOC concentration change to a human generated VOC concentration, resulting in a total human generated VOC concentration (*humanVOC*),
- calculating an estimated $CO_2$ concentration ($eCO_2$) only from the total human generated VOC concentration (*humanVOC*), whereas the estimated $CO_2$ concentration is a part of the total human generated VOC concentration, and the $eCO_2$ concentration is calculated according to $eCO_2 = b \cdot humanVOC,$ whereas b is the ratio between the human generated $CO_2$ and VOC concentration,

wherein the measurement is performed by a VOC sensitive sensor and the splitting, accumulation and calculation is performed by an evaluation processing unit.

By distinguishing the source of the temporal change in the measured VOC concentration it is possible to perform an improved estimation of the human generated $CO_2$ concentration ($eCO_2$). Due to the improved measurement and calculation setup the error of the estimation of $CO_2$ can be reduced.

The method according to the invention further comprises comparing the measured change of the VOC concentration with a previous measurement. This makes it possible to determine the change in the VOC concentration.

If the measured change of the VOC concentration is falling by a ratio x%, both the human generated VOC concentration and the non-human generated VOC concentration is reduced by the same ratio x%. Thereby, x indicates the ratio the measured VOC concentration has changed in a pre-defined time interval. The idea is to recognize different artificial events from the time course of the measured VOC concentration. This is indicated by the ratio x in %, and to react accordingly. If the measured change of the VOC concentration is falling by more than x% a ventilation is detected and both the human generated VOC concentration and the non-human generated VOC concentration is reduced by the same factor/ratio as the measured VOC concentration has been fallen. Ventilation always reduces the human and non-human generated VOC.

If the measured change of the VOC concentration is rising by more than a first threshold, the change of the VOC concentration is added to the non-human generated VOC concentration, otherwise the change of the VOC concentration is added to the human generated VOC concentration. The first threshold is defined by the maximum VOC change rate humans can generate in the room. This depends on the size of the room and the number of humans in the room. Splitting the measured VOC concentration change into human and non-human generated VOC changes allows a better estimation of the human generated $CO_2$.

In accordance with the invention the method further comprises calculating the $eCO_2$ concentration according to $eCO_2 = b \cdot humanVOC,$ whereas $b$ is the ratio between the human generated $CO_2$ and VOC concentration. Thereby the estimated $CO_2$ concentration can be improved by an improved calculation procedure.

If the $eCO_2$ concentration is below a lower limit (LL), the human generated VOC concentration is adjusted to

$$humanVOC = \frac{LL}{b}$$ and $eCO_2$ = LL is reported as $eCO_2$ concentration.

It is known that the natural concentration limit in the earth's atmosphere of $CO_2$ can be in the range of 300ppm and 600ppm, an average value could be 400ppm, therefore the lower limit (LL) is set to a value in the range of 300ppm and 600ppm as an indoor $CO_2$ concentration cannot be lower than 300ppm, but the tendency is rising.

If the $eCO_2$ concentration is above an upper limit (UL), the human generated VOC concentration is adjusted to $$humanVOC = \frac{UL}{b}$$ and $eCO_2$ = UL is reported as $eCO_2$ concentration.

It is known and confirmed that $CO_2$ concentrations above 5000ppm are extremely unlikely in closed rooms or at home, therefore the upper limit (UL) defines also an upper end of a measurement range required by the specific application, and is in the range of 3000ppm and 15000ppm, whereas a UL of 5000ppm can be seen as a maximum indoor $CO_2$ concentration.

If the $eCO_2$ concentration is between the lower limit (LL) and the upper limit (UL), the $eCO_2$ concentration is reported as the true $eCO_2$ concentration according to the above-proposed improved calculation procedure.

In another preferred embodiment the method is performed by a MOX sensor and an evaluation processing unit, whereas the MOX sensor measures the VOC concentration and the evaluation processing unit splits, accumulates, calculates and reports the $eCO_2$ concentration.

In another embodiment a smoothing filter is applied to the measured VOC concentration. This has the advantage to reduce the measurement noise.

The invention will be explained in more detail using exemplary embodiments.

The appended drawings show

Fig. 1    Temporal characteristic of $CO_2$ and VOC concentration generated by a human source;

Fig. 2    Temporal characteristic of $CO_2$ and VOC concentration generated by non-human VOC sources;

Fig. 3    Temporal characteristic of $CO_2$ and VOC concentration, if ventilation happens;

Fig. 4    Temporal characteristic of $CO_2$ and VOC concentration for a real life scenario.

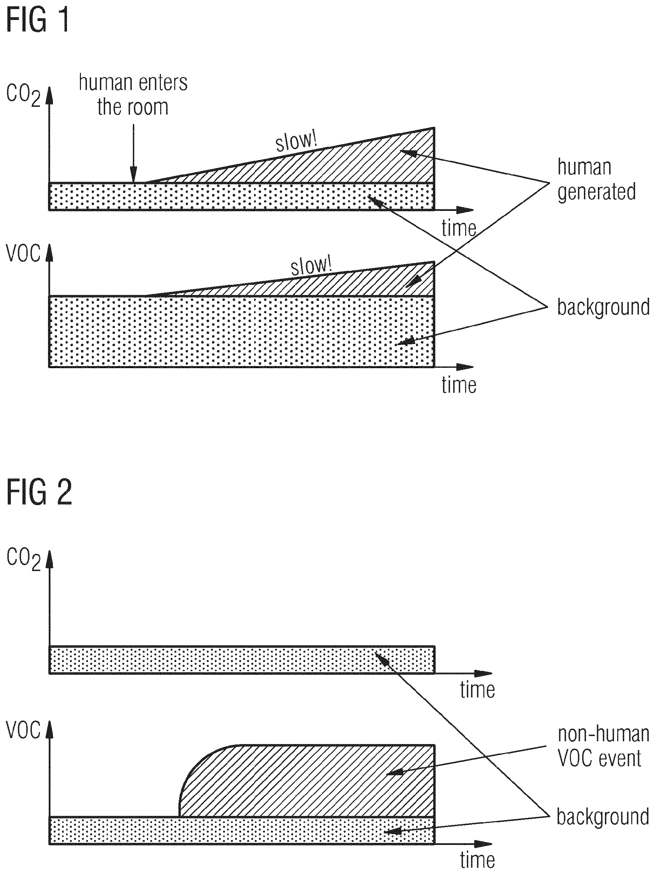

Figure 1 shows the temporal characteristic of the $CO_2$ and VOC concentration indoors if a human enters the room. At first there is a constant $CO_2$ and VOC concentration inside the room. If a human enters the room than the concentrations of $CO_2$ and VOC will increase proportionally but in a very slow way. The slow rise in the $CO_2$ and VOC concentration indicates that the concentration change is generated by a human source.

Figure 2 shows the temporal characteristic of $CO_2$ and VOC concentration generated by non-human VOC sources. At first there is a constant $CO_2$ and VOC concentration inside the room. If the concentration of VOC rises suddenly and the $CO_2$ concentration remains unchanged this indicates that the fast VOC concentration change is caused by a non-human VOC source.

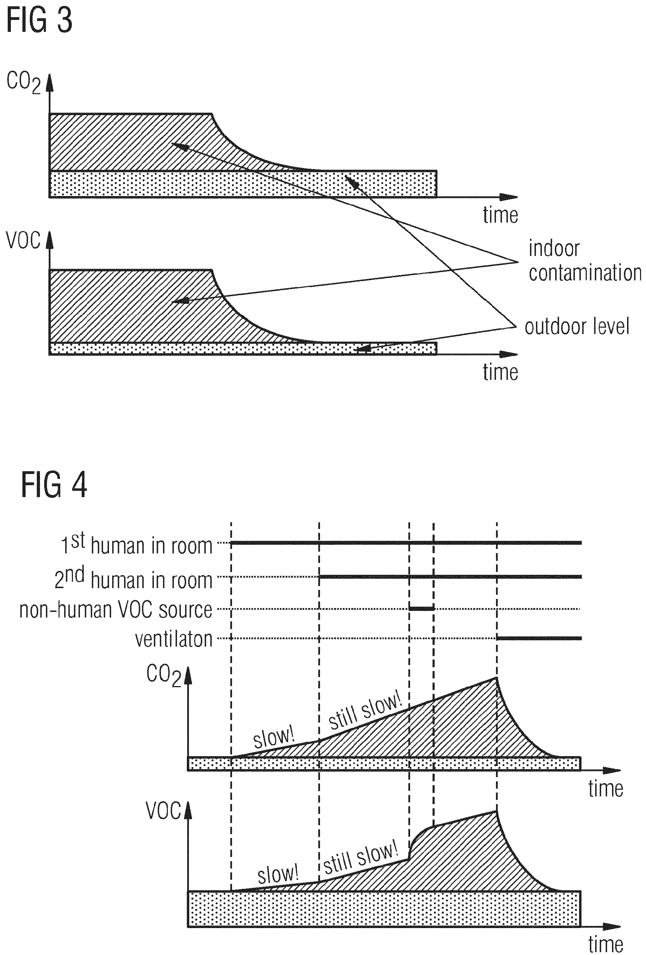

Figure 3 shows the temporal characteristic of $CO_2$ and VOC concentration, if ventilation happens. At first, a high level of $CO_2$ and VOC concentration is present. A fast decrease of both $CO_2$ and VOC concentrations indicate that ventilation is used in order to decrease the $CO_2$ and VOC concentrations simultaneously.

Figure 4 shows the temporal characteristic of $CO_2$ and VOC concentration for a real life scenario. The lines at the top of the figure mark the duration of an object which stays in a room. In this case, the objects examined in the figure are a first human, a second human and an artificial VOC source, as well as a ventilation event. According to the presence of the different objects or events in the room the diagrams show the process of the $CO_2$ concentration and the VOC concentration in that room. If non object, neither a human nor a non-human source is inside the room, a constant concentration of $CO_2$ and VOC will be detected in the room. If a first human enters the room the $CO_2$ as well as the VOC concentration will be increased slowly. If a second additional human enters the room the concentration of $CO_2$ as well as VOC increase further but still in a slow way. If a non-human VOC event occurs in the room there will be detected a fast increase in VOC concentration, but the increase in $CO_2$ concentration remains constant with the same ration as if no non-human VOC event has been occurred, because the non-human event did not add $CO_2$. If the non-human VOC event ends the concentration of $CO_2$ as well as VOC increase further but in a slow way as before the non-human VOC event. If a ventilation is used both the concentration of $CO_2$ as well as VOC decrease quickly.

**Claims**

1.  A method to improve estimated $CO_2$ ($eCO_2$) concentration for indoor applications, comprising the following steps:

    - observing a temporal change of a VOC concentration by measuring a present VOC concentration and comparing the result with a previous VOC concentration of a previous measurement of the VOC concentration,
    - splitting the change of the VOC concentration into a human generated VOC concentration change and a non-human generated VOC concentration change,
    - accumulate the human generated VOC concentration change to a human generated VOC concentration, resulting in a total human generated VOC concentration (*humanVOC*),
    - calculating an estimated $CO_2$ concentration ($eCO_2$) only from the total human generated VOC concentration, whereas the estimated $CO_2$ concentration is a part of the total human generated VOC concentration, and the $eCO_2$ concentration is calculated according to $eCO_2 = b \cdot humanVOC$, whereas b is the ratio between the human generated $CO_2$ and VOC concentration,

    wherein the measurement is performed by a VOC sensitive sensor and the splitting, accumulation and calculation is performed by an evaluation processing unit.

2.  The method according to claim 1, wherein if the measured change of the VOC concentration is falling by a ratio x%, reduce both the human generated VOC concentration and the non-human generated VOC concentration by the same ratio x%.

3.  The method according to claim 1, wherein if the measured change of the VOC concentration is rising by more than a first threshold, add the change of the VOC concentration to the non-human generated VOC concentration, otherwise add the change of the VOC concentration to the human generated VOC concentration.

4. The method according to claim 1, wherein if the eCO$_2$ concentration is below a lower limit (LL), adjust the human generated VOC concentration to $humanVOC = \frac{LL}{b}$ and report eCO$_2$ = LL.

5. The method according to claim 4, wherein LL is in the range of 300ppm to 600ppm.

6. The method according to claim 1, wherein if the eCO$_2$ concentration is above an upper limit (UL), adjust the human generated VOC concentration to $humanVOC = \frac{UL}{b}$ and report eCO$_2$ = UL.

7. The method according to claim 6,

   wherein UL is an upper
   end of a measurement range required by the application, and is in the range of 3000ppm and 15000ppm.

8. The method according to one of the former claims, wherein if the eCO$_2$ concentration is between the lower limit (LL) and the upper limit (UL), report the eCO$_2$ concentration.

9. The method according to one of the former claims, wherein a smoothing filter is applied to the measured VOC concentration.

**Patentansprüche**

1. Verfahren zum Verbessern der geschätzten CO$_2$-Konzentration (eCO$_2$) für Innenraumanwendungen, das die folgenden Schritte umfasst:

   - Beobachten einer zeitlichen Änderung einer VOC-Konzentration durch Messen einer aktuellen VOC-Konzentration und Vergleichen des Ergebnisses mit einer früheren VOC-Konzentration einer früheren Messung der VOC-Konzentration,
   - Aufteilen der Änderung der VOC-Konzentration in eine vom Menschen hervorgerufene Änderung der VOC-Konzentration und eine nicht vom Menschen hervorgerufene Änderung der VOC-Konzentration,
   - Akkumulieren der Änderung der vom Menschen hervorgerufenen VOC-Konzentration zu einer vom Menschen hervorgerufenen VOC-Konzentration, was zu einer gesamten vom Menschen hervorgerufenen VOC-Konzentration (humanVOC) führt,
   - Berechnen einer geschätzten CO$_2$-Konzentration (eCO$_2$) nur aus der gesamten vom Menschen hervorgerufenen VOC-Konzentration, wobei die geschätzte CO$_2$-Konzentration ein Teil der gesamten vom Menschen hervorgerufenen VOC-Konzentration ist und die eCO$_2$-Konzentration gemäß $eCO_2 = b \cdot humanVOC$ berechnet wird, wobei b das Verhältnis zwischen der vom Menschen hervorgerufenen CO$_2$- und VOC-Konzentration ist, wobei die Messung durch einen VOC-empfindlichen Sensor durchgeführt wird und das Aufteilen, Akkumulieren und Berechnen durch eine Auswerteeinheit durchgeführt wird.

2. Verfahren nach Anspruch 1, wobei, wenn die gemessene Änderung der VOC-Konzentration um ein Verhältnis x % abnimmt, sowohl die vom Menschen hervorgerufene VOC-Konzentration als auch die nicht vom Menschen hervorgerufene VOC-Konzentration um dasselbe Verhältnis x % verringert werden.

3. Verfahren nach Anspruch 1, wobei, wenn die gemessene Änderung der VOC-Konzentration um mehr als einen ersten Schwellenwert ansteigt, die Änderung der VOC-Konzentration zu der nicht vom Menschen hervorgerufenen VOC-Konzentration addiert wird, andernfalls wird die Änderung der VOC-Konzentration zu der vom Menschen hervorgerufenen VOC-Konzentration addiert.

4. Verfahren nach Anspruch 1, wobei, wenn die eCO$_2$-Konzentration unter einem unteren Grenzwert (LL) liegt, die vom Menschen hervorgerufene VOC-Konzentration an $humanVOC = \frac{LL}{b}$ angepasst und eCO$_2$ = LL gemeldet wird.

**5.** Verfahren nach Anspruch 4, wobei LL im Bereich von 300 ppm bis 600 ppm liegt.

**6.** Verfahren nach Anspruch 1, wobei, wenn die $eCO_2$-Konzentration über einem oberen Grenzwert (UL) liegt, die vom

$$humanVOC = \frac{UL}{b}$$

Menschen hervorgerufene VOC-Konzentration an $\qquad$ angepasst und $eCO_2$ = UL gemeldet wird.

**7.** Verfahren nach Anspruch 6, wobei UL ein oberes Ende eines für die Anwendung erforderlichen Messbereichs ist und im Bereich von 3000 ppm und 15000 ppm liegt.

**8.** Verfahren nach einem der vorhergehenden Ansprüche, wobei, wenn die $eCO_2$-Konzentration zwischen dem unteren Grenzwert (LL) und dem oberen Grenzwert (UL) liegt, die $eCO_2$-Konzentration gemeldet wird.

**9.** Verfahren nach einem der vorhergehenden Ansprüche, wobei ein Glättungsfilter auf die gemessene VOC-Konzentration angewendet wird.

**Revendications**

**1.** Procédé servant à améliorer une concentration estimée de $CO_2$ ($eCO_2$) pour des applications intérieures, comprenant les étapes suivantes :

- observation d'une variation temporelle d'une concentration de COV par mesure d'une concentration actuelle de COV et comparaison du résultat avec une concentration précédente de COV d'une mesure précédente de la concentration de COV,
- séparation de la variation de la concentration de COV en une variation de concentration de COV générée par l'homme et une variation de concentration de COV non générée par l'homme,
- accumulation de la variation de concentration de COV générée par l'homme en une concentration de COV générée par l'homme, pour obtenir une concentration totale de COV générée par l'homme (*humanVOC*),
- calcul d'une concentration estimée de $CO_2$ ($eCO_2$) uniquement à partir de la concentration totale de COV générée par l'homme, alors que la concentration estimée de $CO_2$ est une partie de la concentration totale de COV générée par l'homme, et la concentration $eCO_2$ est calculée selon $eCO_2 = b \cdot humanVOC$, où *b* est le rapport entre les concentrations de $CO_2$ et de COV générées par l'homme,

dans lequel la mesure est effectuée par un capteur sensible aux COV et la séparation, l'accumulation et le calcul sont effectués par une unité de traitement d'évaluation.

**2.** Procédé selon la revendication 1 dans lequel, si la variation mesurée de la concentration de COV baisse d'une proportion x%, la concentration de COV générée par l'homme et la concentration de COV non générée par l'homme sont toutes deux réduites dans la même proportion x%.

**3.** Procédé selon la revendication 1 dans lequel, si la variation mesurée de la concentration de COV monte de plus d'un premier seuil, la variation de la concentration de COV est ajoutée à la concentration de COV non générée par l'homme, sinon la variation de la concentration de COV est ajoutée à la concentration de COV générée par l'homme.

**4.** Procédé selon la revendication 1 dans lequel, si la concentration $eCO_2$ est au-dessous d'une limite inférieure (*LL*),

la concentration de COV générée par l'homme est ajustée à $humanVOC = \frac{LL}{b}$ et $eCO_2$ = *LL* est signalé.

**5.** Procédé selon la revendication 4, dans lequel *LL* se situe dans la gamme de 300 ppm à 600 ppm.

**6.** Procédé selon la revendication 1 dans lequel, si la concentration $eCO_2$ est au-dessus d'une limite supérieure (*UL*),

la concentration de COV générée par l'homme est ajustée à $humanVOC = \frac{UL}{b}$ et $eCO_2$ = *UL* est signalé.

**7.** Procédé selon la revendication 6, dans lequel *UL* est une extrémité supérieure d'une gamme de mesure requise par l'application, et se situe dans la gamme de 3000 ppm à 15 000 ppm.

**8.** Procédé selon une des revendications précédentes dans lequel, si la concentration $eCO_2$ se situe entre la limite inférieure (*LL*) et la limite supérieure (*UL*), la concentration $eCO_2$ est signalée.

**9.** Procédé selon une des revendications précédentes, dans lequel un filtre de lissage est appliqué à la concentration mesurée de COV.

## FIG 1

## FIG 2

FIG 3

FIG 4

**EP 3 495 813 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6344798 B